# EUROPEAN PATENT APPLICATION

(11) **EP 2 120 170 A1**
(43) Date of publication of application: **18.11.2009**
(21) Application number: 09006472.6
(22) Date of filing: 13.05.2009
(51) Int. Cl.: G06F 19/00

(54) **Distributed integrated image data management system**

(30) Priority: 14.05.2008 US 71709 P
(71) Applicant: Algotec Systems Ltd., 43107 Raanana (IL)
(72) Inventor: Benjamin, Menashe, 43555 RaAnana (IL); Bauer, Ori, 55525 Kiryat-Ono (IL); Velan, Noam, 45296 Hod-HaSharon (IL)
(74) Representative: Becker Kurig Straus

(57) **Abstract**

A system and method for increasing integration within and between medical sites with medical information systems, optionally using a single device which is suitable for multiple sites. In some embodiments, the device forms a network where devices can exchange data across networks. In some embodiments, the device provides and supports upgrading of hospital capability.

## Description

### RELATED APPLICATIONS

This application is related to U.S. Provisional Patent Application, Attorney Docket No. 44045, entitled "METHODS, SYSTEMS AND A PLATFORM FOR MANAGING MEDICAL DATA RECORDS", filed on even date as this application, the disclosure of which is incorporated herein by reference.

### FIELD AND BACKGROUND OF THE INVENTION

The present invention, in some embodiments thereof, relates to medical image management and, more particularly, but not exclusively, to a system for integrating medical imaging across locations and platforms.

Computerization of hospital systems has traditionally been incremental in manner. Over the years, various computer systems have been linked together. Quite often, however, a hospital includes computer systems form multiple vendors. One reason is that various hardware and/or human systems are provided with their own computerized systems (e.g., image processing workstation can come with an imaging system). There is a gradual movement to integrate such systems, for example, by setting standards (DICOM, HL7) and by providing integrated systems that replace several hospital systems simultaneously. DICOM is a transmission protocol that creates, stores, prints, and forwards image data to and from the imaging modalities and PACS. HL7 identifies patients, processes orders, and stores reports, but it cannot manage DICOM data.

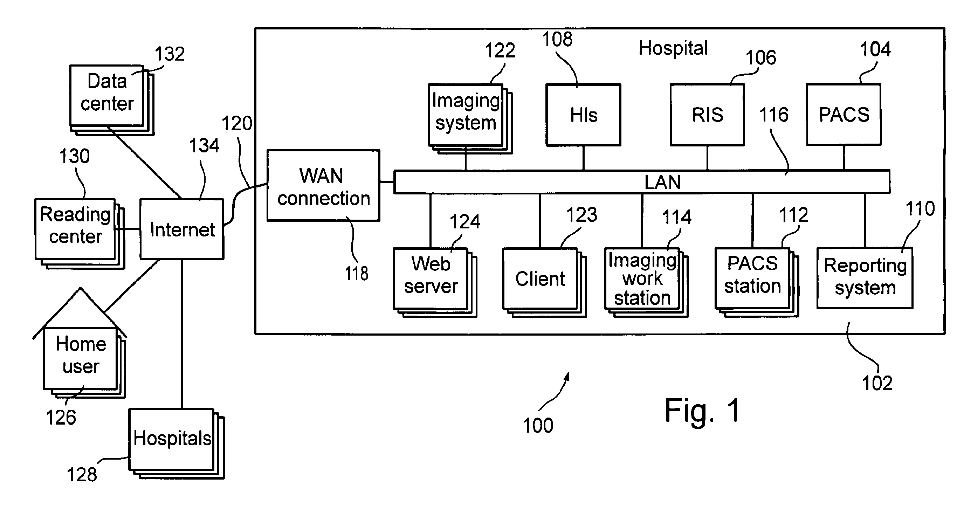

Fig. 1 shows a health care system 100 including an exemplary hospital 102 with multiple computer systems, each by a different vendor, in addition to one or more imaging systems 122. Many hospitals have some or all of these systems, each possibly by a different vendor:
A PACS 104 - a system for managing radiological images and image retrievals.
A RIS 106 - a system for storing information regarding radiological studies. More commonly, the RIS is provided by a same vendor and in compatibility with PACS.
A HIS 108 - a system for storing patient and hospital information. The HIS is often integrated with the RIS.
A Reporting system 110 - a system for a physician to enter and publish radiological reports, can include a voice recognition feature.
A PACS compatible work station (optionally multiple) 112 - a station which can connect to a PACS system and can be used to process images, for diagnosis.
A Processing workstation (optionally multiple) 114 - an image processing station used for specialized processing of some types of images. Typically provided separately from a PACS workstation.
One or more clients 123 that can connect (in a no-integrative manner) to various hospital systems are typically provided, often with different interfaces.
In spite of the variety, hospitals typically share a single networking backbone 116 and/or connection 118 to the outside world, for example to an internet 134, via a link 120. Some data may be stored and/or managed at a data center 132.
In addition, hospitals often include servers for remote access, for example for reviewing radiological studies from home 126 (one or more) or by a reading group 130 (one or more); for example, such systems may be supported by one or more web servers 124 (or a single web server with multiple applications executing). In addition, remote access and reporting to outside the hospital often uses multiple methods, such as telephone, electric text messages (e.g., beepers), fax and e-mail.
It should be appreciated that each hospital may have a different set of vendors. Additional computerizing systems used include data centers 134 which manage data from multiple hospitals 128 and systems used by radiological reading groups, which contain data related to multiple hospitals.

### SUMMARY OF THE INVENTION

A broad aspect of some embodiments of the invention relates to a unified design for an integration system unit which can be easily installed in a variety of different site configurations including various clinical/computerization setups and which links the sites to provide integrated functionality.

There is provided in accordance with an exemplary embodiment of the invention, a method of linking together a plurality of medical sites, selected as one or more instances of one or more elements of the following group of an imaging site, a hospital, a clinic, a reading center and a data center, comprising:
(a) installing an integration device at each of the sites;
(b) linking at least one integration device to a plurality of disparate medical information systems at one of the sites, to allow access to data therefrom; and
(c) exposing said data from said one integration device via the other integration device to a consumer of the data.

In an exemplary embodiment of the invention, exposing comprises reading and updating.

In an exemplary embodiment of the invention, said linking comprises collecting meta data regarding said data at said one integration device.

In an exemplary embodiment of the invention, said linking comprises receiving at least an indication of said data without being allowed direct access to said data.

In an exemplary embodiment of the invention, the method comprises diagnosing on a single worklist and workstation studies from said plurality of sites by linking to one of said integration devices.

In an exemplary embodiment of the invention, at least two of said disparate systems are incompatible with each other.

In an exemplary embodiment of the invention, said data comprises radiological imaging data and one of said medical information systems is a PACS system.

There is provided in accordance with an exemplary embodiment of the invention, a method of upgrading a hospital information system infrastructure, comprising:
(a) installing an integration device in the hospital;
(b) linking said device to a plurality of hospital information systems and collecting at least meta data on data stored therein; and
(c) accessing data on a plurality of said systems via said integration device.

In an exemplary embodiment of the invention, the method comprises providing reliability redundancy using said integration device.

In an exemplary embodiment of the invention, the method comprises installing at least one additional integration device which is at least partially functionally redundant with said integration device.

In an exemplary embodiment of the invention, the method comprises adding functionality of a type associated with one of said systems using said device.

In an exemplary embodiment of the invention, the method comprises gradually taking over at least one of said systems using said device.

In an exemplary embodiment of the invention, said system is a RIS or a PACS or both.

In an exemplary embodiment of the invention, the method comprises recovering from a failure using said device and one or both of meta data and data stored thereon.

In an exemplary embodiment of the invention, accessing comprises integrating data from multiple systems.

In an exemplary embodiment of the invention, the method comprises changing a workflow in a site of said infrastructure by configuring said integration device.

In an exemplary embodiment of the invention, the method comprises data mining data across said systems.

In an exemplary embodiment of the invention, the method comprises data mining data across sites by combining data via said integration device and an integration device at another site.

There is provided in accordance with an exemplary embodiment of the invention, a method of managing a radiological reading group, comprising:
(a) collecting studies from a plurality of sites not on a shared PACS system;
(b) arranging said studies into a single worklist;
(c) managing said worklist across a plurality of readers located at a plurality of sites using an online computer system.

In an exemplary embodiment of the invention, the method comprises automatically updating said list within a time frame of less than 15 minutes.

In an exemplary embodiment of the invention, the method comprises accessing and diagnosing a study at a site not affiliated with the study, in response to said worklist.

In an exemplary embodiment of the invention, the method comprises tracking availability of said readers using a computer.

There is provided in accordance with an exemplary embodiment of the invention, a method of radiological diagnosis, comprising:
(a) diagnosing a study; and
(b) generating a report on a same online computer system as includes a RIS and a PACS.

In an exemplary embodiment of the invention, said generating comprises automatically facilitating said generating using data form said RIS.

In an exemplary embodiment of the invention, said online computer system links a referring physician, a reader and a reporting system.

There is provided in accordance with an exemplary embodiment of the invention, an integration device, comprising:
(a) a remote access module, configured to support remote clients;
(b) a data management module configured to manage data on the device and off of the device;
(c) at least one of a PACS functionality and a RIS functionality; and
(d) an integration module configured to at least one of link said device to a plurality of disparate hospital systems and link said device to an integration device at an additional site, for data sharing therewith.

In an exemplary embodiment of the invention, said remote access module installs a complete suite of medical image processing and reporting software on a client device.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

In addition, where a single computer is suggested, a plurality of linked computers may be used in some circumstances. Multiple functionalities may be implemented on a single or multiple computers.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Fig. 1 is a schematic diagram showing multiple vendors in a prior art hospital computer configuration;
Fig. 2 is a schematic diagram showing a hospital computer configuration including one or more integration devices in accordance with an exemplary embodiment of the invention;
Fig. 3 is a schematic diagram showing parts of an integration device in accordance with an exemplary embodiment of the invention;
Figs. 4A and 4B are a flowchart of a method of using a hospital integration system in accordance with an exemplary embodiment of the invention, for a process of imaging and reporting;
Fig. 5 is a flowchart of a method of work planning and image diagnosis across hospital networks, in accordance with an exemplary embodiment of the invention;
Fig. 6 is a flowchart of a method of retrofitting an existing hospital network, in accordance with an exemplary embodiment of the invention; and
Fig. 7 is a flowchart of a method of image reading from a remote location, in accordance with an exemplary embodiment of the invention.

### DESCRIPTION OF EMBODIMENTS OF THE INVENTION

### Overview

The present invention, in some embodiments thereof, relates to medical image management and, more particularly, but not exclusively, to a system for integrating medical imaging across locations and platforms.

Referring back to Fig. 1, it may be appreciated that a lack of integration between the various hospital systems and/or personnel may cause a reduction in efficiency. In an exemplary embodiment of the invention, a system and method for integration of hospital and human infrastructure is provided. Optionally, the infrastructure utilizes a single design and a single interface.

In an exemplary embodiment of the invention, an integration device includes a single unit, which includes all the functionality needed to integrate with (e.g., via emulation, message interception, message addressing and/or connection to ports) a variety of hospital computerization systems/information systems and all the functionalities needed to integrate with other, similar integration devices in other hospitals and thereby provide a multi-site network. In an exemplary embodiment of the invention, the unit also supports local functionalities, such as data processing and replacement of one or more hospital systems functions.

Optionally, support for acting as a server to client stations is provided. Optionally or alternatively, support for data archiving and management is provided. Optionally or alternatively, support for workflow management is provided. Optionally or alternatively, support for reporting is provided. Optionally or alternatively, support for diagnosis is provided, for example, 3D display and processing.

In an exemplary embodiment of the invention, the integration provided by the integration device comprises allowing and supporting data and/or instruction flow between disparate systems and/or locations.

In an exemplary embodiment of the invention, the integration provided by the integration device comprises carrying out processes using a plurality of systems acting in concert.

It is a particular feature of some embodiments of the invention, that integration of processes across systems can yield significant gains in efficiency and/or reduce waiting time. In particular, each user can, in some embodiments, receive the data s/he needs, where he needs it and when he needs it, even if the data originates from multiple different systems by different vendors.

Optionally or alternatively, the integration allows better control over the system processes and a better process of diagnosis to be achieved. Optionally or alternatively, the integration of information and control allows implementing processes not previously practical to implement, for example, workload distribution, efficiency monitoring, real-time messaging, conferencing and procedure efficacy tracking.

Optionally or alternatively, the integration is useful and/or improves productivity and/or reduces error by reducing the number of different user interfaces a user must learn (desirably to one).

In an exemplary embodiment of the invention, there is provided an inter-hospital network having an installation and usage that can be substantially transparent to any particular hospital (or other node). In an exemplary embodiment of the invention, the network is used to pass data between hospitals, for example, as an aid in diagnosis and/or for supporting more complete patient records. In an exemplary embodiment of the invention, the network does not require substantial reprogramming of existing systems. Rather, an add-on integration device (optionally a single box) is provided in each hospital and these devices attend to mapping and exchanging data, as desired. In some cases, existing hospital systems are set up to expose data and/or communications to the integration devices.

In an exemplary embodiment of the invention, there is provided an intra-hospital integration system which support aggregation and exchange of information between different systems in a hospital, optionally serving as a gateway to outside hospitals and/or other data user sand/or producers.

In an exemplary embodiment of the invention, there is provided an integration device which links together medical systems by emulating the interface and/or a user of two different medical information systems. Optionally, the integration is used not only for reading information but also for writing information.

In an exemplary embodiment of the invention, a single device architecture is used for multiple, optionally linked, different installations. Optionally, a same software is used, with a difference between installations being in number of processors, size of processor, memory and/or storage. However, optionally, the devices are interchangeable with regard to function. In an exemplary embodiment of the invention, the use of a single architecture simplifies set up, training (e.g., due to interface uniformity) and/or maintenance of the system and network created using the devices.

In an exemplary embodiment of the invention, the single architecture supports one or more of:
(a) a single server, for web, storage and workflow infrastructure;
(b) a single display and processing infrastructure
(c) a single image and report distribution infrastructure
(d) a uniform user interface across users, systems and uses; and
(e) a single base for customization for a wide range of uses.

In an exemplary embodiment of the invention, the integration process is simple and comprises basically of physically installing the integration device in one or more hospitals or other usage locations and a relatively simple set-up.

In an exemplary embodiment of the invention, the integration device as described herein is used to support data mining applications in which the data collected about a plurality of radiological-related processes, patients, users, imagers, sites and/or costs are analyzed to detect useful trends and/or problems.

In an exemplary embodiment of the invention, the workflow of study diagnosis can now be managed by the reading group or by the hospital, allowing previous bottle necks to be overcome. Such bottle necks, for example, interfered with the ability to work form one site on studies of another site and/or interfered with the ability to monitor and assign and cooperate on work when readers are not all at a same location.

It should be noted that in accordance with some embodiments of the invention, an integration device does not serve as a gateway between sites, but rather as a separate network which has access to data at the sites. In other embodiments/configurations, integration device does serve as a gateway in that it exposes and allows manipulation of data at one site under one system to a person or system at a different site and possible using a non-native interface.

In an exemplary embodiment of the invention, there is provided a method of upgrading an installation (e.g., a hospital), while also providing additional ability for later updating, in which an integration device is provided and thereafter, new functionalities can be provided while using legacy systems and/or gradually and/or abruptly taking over from them. Optionally or alternatively, ability and/or robustness may be improved by increasing the number of integration devices.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Location

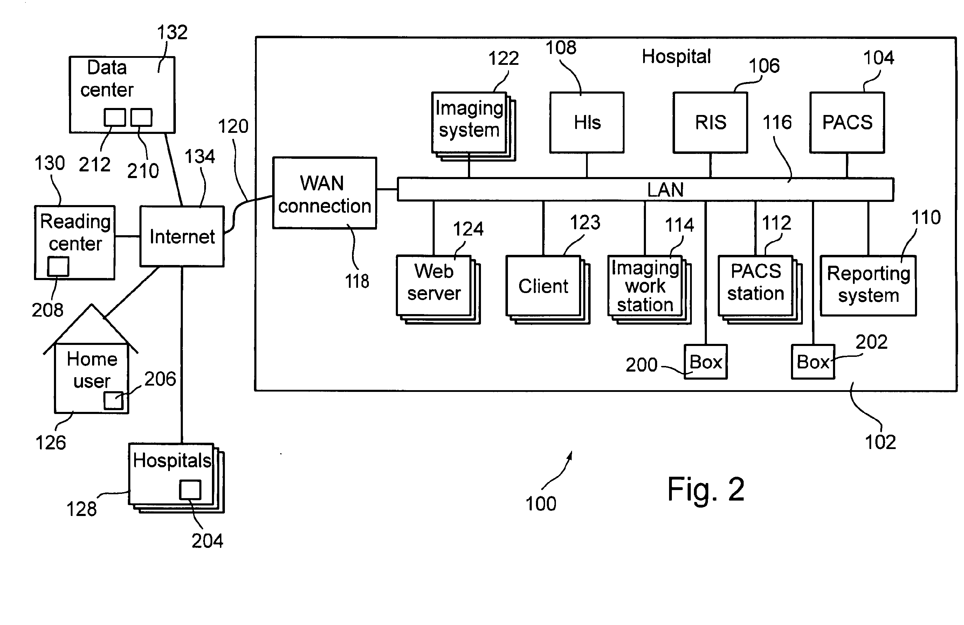

Fig. 2 is a schematic diagram showing a hospital computer configuration including a plurality of possible locations for integration devices in accordance with an exemplary embodiment of the invention.

In an exemplary embodiment of the invention, an integration device 200 is connecting to LAN 116 inside a hospital 102. Optionally, one or more additional integration devices 202 are connected on the same LAN. Optionally or alternatively to LAN 116, at least some hospital information systems, such as a RIS or an integration device are connected by other means, such as a WAN.

In an exemplary embodiment of the invention, one or more integration devices 204 are provided at other hospitals and act together as a network. While the network as shown as passing through an internet, this is not essential and the network may include other types of data links, instead or in addition. In an exemplary embodiment of the invention, one or more integration devices 210 are provided at a data center, which may store and/or mage data for a plurality of hospital. In some cases, a device 212 with different functionality is provided at the data center. For example, the load of the work between the devices may be balanced, optionally with one device being master and one slave. In another example, one of the devices is configured or provided as a RIS system.

In an exemplary embodiment of the invention, an integration device 208 is provided at a reading center, for example, being used for image processing, diagnosis and/or workflow control.

In an exemplary embodiment of the invention, an integration device 206 is provided at a home. Optionally, a home user acts as a client that connects to an integration device (e.g., 208), however, in some cases, local processing ability is desirable. Typically, a home (or clinic) integration device will have reduced processing power and/or storage.

### Exemplary architecture

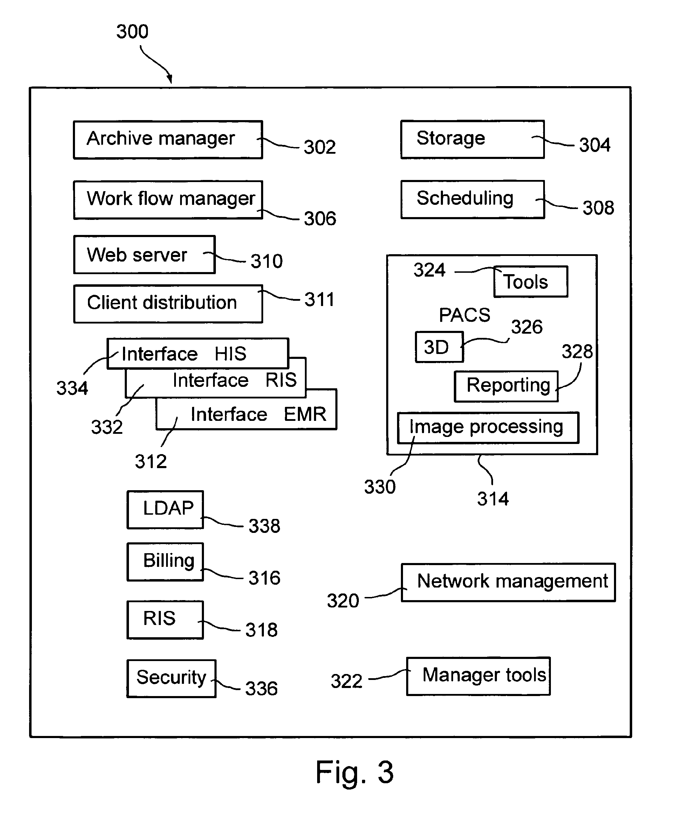

Fig. 3 is a schematic block diagram showing various hardware and/or software modules, some or all of which are provided in an integration device 300 (which can be located, for example, as described with respect to Fig. 2), in accordance with an exemplary embodiment of the invention.

### Archive manager and storage

In an exemplary embodiment of the invention, device 300 includes an archive manager 302, which manages data stored at the site of the device, for example, data stored on the device at clients and/or in hospital data systems and storage devices. In an exemplary embodiment of the invention, archive manager 302 maintains meta-data on data it manages, for example, image identifiers and statuses. Optionally, data is managed in a multi-tier scheme, for example, data may be in fast storage (e.g., an online storage 304 of the device), available storage (e.g., stored on a hospital database). Slow storage (e.g., on a tape device) or off-line, for example, on DVDs, available locally or off-site.

In an exemplary embodiment of the invention, archive manager 302 also interacts with other integration devices, to manage data across the hospital network, for example, including, meta-data on data in other hospitals and/or integration devices or data centers.

In an exemplary embodiment of the invention, archive manager 302 includes a database of data locations, where the field can be local (e.g., physical storage on integration device 300) or remote.

In an exemplary embodiment of the invention, archive manager 302 provides a pre-fetch function whereby data which will be needed is brought to a more available status, for example, by retrieving it from a slow and/or remote storage.

In an exemplary embodiment of the invention, archive manager 302 provides a data routing function, whereby data is retrieved via available and/or alternate routes. For example, if an internet connection to a remote data source (e.g., another hospital) is down, data may still be retrieved via an intermediary integration device that has live connections to the source and destination, albeit via a slower connection. In some cases, data which is less reliable (e.g., from a backup source) may be provided, with a suitable indication, if newest data is not available.

In an exemplary embodiment of the invention, for each real destination there is a mapping which states the next hop on how to get (the data) to that destination. In one implementation the next hop will be the final destination. In other implementations, the next hop is set up to be some other server which will then forward the request to the destination (or to another intermediate server). Optionally, the destination is pinged on a timely basis, and if the connection fails - the next hop for that destination is changed to be some common datacenter which knows how to reach all the other destinations, including the desired one.

In an exemplary embodiment of the invention, archive manager 302 is used to plan and perform backups of data. Optionally or alternatively, other lifecyle activities are carried out, for example, one or more of:
(a) managing more then one copy of the data for redundancy;
(b) deleting data after a predefine period of time as dictated by local legislation;
(c) managing a study availability (e.g., how quickly it will load) by various parameters (such as study age, and procedure type); and
(d) managing hardware obsolescence by migrating an entire TIER residing on old hardware to a new one.

In an exemplary embodiment of the invention, archive manager 302 can operate in a mode of partial backup, in which data that is lost from hospital systems due to a system failure, is provided instead by archive manager 302, from local or remote copies of the data.

In one example, if the main PACS is lost or temporarily down, the local workstations query the integration device directly until the PACS is back online. In another example, if a local PACS storage is permanently lost, integration device life cycle functions are used to restore the local PACS by copying everything that is available, either locally of from other integration devices in the workflow grid.

In an exemplary embodiment of the invention, data management is according to what is described in the above related application, the disclosure of which is incorporated herein by reference.

In an exemplary embodiment of the invention, data is transferred between integration devices using DICOM or using a proprietary process, for example, as described in the above application.

### Workflow manager

In an exemplary embodiment of the invention, device 300 includes a workflow manager 306, which assigns studies to the radiologists who will diagnose them and manages the worklists of the site and/or of other sits connected to the network of integration devices. In an exemplary embodiment of the invention, this scheduling (described in some more detail below) uses automatic rules and/or a user interface to divide up work, optionally with user intervention and/or decision, for example, by a workload manager.

In an exemplary embodiment of the invention, worklists may be exported to other sites and/or other reading groups. For example, two reading groups may be in charge of diagnosing studies in a same site, with integration device 300 coordinating between the workgroups.

In an exemplary embodiment of the invention, a worklist includes a hierarchical (or other) arrangement of worklists between sites, groups of sites and reading groups. Optionally, the arrangement defines rules for passing work between sites, optionally including rules for money collection. Optionally, the costs for diagnosis are different for different situations (group, study, urgency, load on group) and the workflow manager includes a user interface suggesting what costs for an actual work transfer would be and/or an automatic system (e.g., based on optimization or rules) for distributing work, optionally to reduce work, achieve a desired turn around and/or other considerations and/or tradeoffs.

### Scheduling

In an exemplary embodiment of the invention, device 300 includes a scheduling module 308 which generates schedules for radiologists and/or imaging stations and/or books when a patient will come to be examined, which Modality will do the scan and/or what type of protocol will be performed. Optionally, for example as described below, the schedules are continuously updated, for example, as new needs arrive and/or resource availability is updated.

### Web server

In an exemplary embodiment of the invention, device 300 includes a web server 310, which is used for supporting remote clients (e.g., for home diagnosis). Optionally, the server also provides software to the client stations, for example, as ActiveX applets. In some cases, a web server is also used for handling client stations on the same LAN, for example, in the hospital. In an exemplary embodiment of the invention, web server 310 also attends to data streaming and preprocessing (e.g., compression, layers), for such streaming.

Optionally or alternatively, web server 310 serves as a portal for administration tools (e.g., for remote administration of integration device 300 and/or for access to various administration functionalities of integration device 300.

Optionally or alternatively, web server 310 provides IHE integrations via web services (e.g., WADO, XDS).

### Client distribution

In an exemplary embodiment of the invention, a separate client distribution component 311 is provided. Optionally, this component is used to provide client software, client patches and/or client upgrades (e.g., by querying client software and suggesting or providing an upgrade when needed and/or available). Optionally, the components provide and/or create and configure a client software for download. Optionally, such configuration can depend on the abilities of the client machine and/or bandwidth. Optionally, such configuration is via a configuration file attached to software to be downloaded. Optionally or alternatively, the configuration includes a division of labor between integration device 300 and the client system. In an exemplary embodiment of the invention, such download is provided by generating a link to an ActiveX component to be followed via the web server.

### Interfaces

In an exemplary embodiment of the invention, device 300 includes interfaces to one or more of a RIS (332), HIS (334) and/or EMR (Electronic Medical Record) (312) systems. In an exemplary embodiment of the invention, the interfaces are used for accepting data from such systems and/or send data there to. For example, data can be received via messages or sent by emulating a client station with a user operating on it. In some cases, such emulation is provided as a set of available emulators (e.g., per software vendor, type and/or version) which are selected upon installation and/or configuration and/or downloaded if needed.

In an exemplary embodiment of the invention, the interfaces are provided as sets of alternative interfaces, each designed for a different version and/or vendor of the system being interfaced with. In an exemplary embodiment of the invention, device 300 is provided with a complete set of such interfaces and/or additional interfaces may be downloaded automatically as needed. In an exemplary embodiment of the invention, this allows device 300 to be integrated in a plug-and-play manner, by which device 300 identifies the systems in a hospital and activates interfaces for those systems, with minimal user involvement (e.g., user needing only to accept system recommendation or select between a small number of provided choices).

### Billing

In an exemplary embodiment of the invention, device 300 includes a billing module 316 that prepares bills, as known in the art, for example, and/or associates a set of data indicating the procedure performed, with a bill prepared locally and/or off site. Such association may assist in collection and/or other justification of the bill. Optionally or alternatively, the billing module is used to collect cost information, for example, for later data mining.

### RIS

In some embodiments of the invention, device 300 includes a fully functional or partly functional RIS system 318. Optionally, device 300 is first used with an existing RIS and after a while, RIS 318 is used instead. In an exemplary embodiment of the invention, this is performed by configuration of the systems at the site. For example: A Modalities system will read the modality worklist of 318 and a HIS will direct all HL7 messages to RIS 318 instead of out of to the old RIS.

### PACS

In an exemplary embodiment of the invention, device 300 includes a full-featured PACS station 314, with the particular feature that at least 90% of any image processing (e.g., using an image processing module 330) and diagnosis by a user may be performed using tools 324, thereby allowing a user to remain in his seat. Optionally, 3D visualization tools 326 are provided. Optionally, a built-in reporting module 328, or an interface to existing systems, is provided.

In an exemplary embodiment of the invention, the integration provided by PACS 314 and/or the connection to various hospital systems allow the automation and/or other enhancement of various processes, such as diagnosis and/or reporting, for example, as described below. Additional exemplary processes which can be enhanced/automated, include, one or more of automatic assigning of studies to radiologists and automatic distribution of results to the referring physician (e.g., via email, FAX).

In some cases, a radiologist works directly on device 300. In other cases, the radiologist works as a client of device 300, with processing being done at the client. In other cases, the processing is performed on integration device 300 and the client views it remotely. As noted above, some of the processing may be local to the client and some on integration device 300.

With regard to reporting, entry is typically at client. Processing of the entry may be locally, at integration device 300 and/or in a combination.

### Network management

In an exemplary embodiment of the invention, device 300 includes a network management module 320, which manages the relationship between the various integration device sin the network. As noted above, there may be a routing table (e.g., for a grid of sites connected by integration devices), which is optionally managed by the network management. Optionally or alternatively, the module also links to remote sites without a integration device 300, for example, using DICOM and HL7 configuration.

In general, it is noted that a significant portion of the data on the sites and/or grid may not be under the control of the integration devices, but is managed using meta data and/or is accessible via integration device 300.

It should be noted that in some cases, a single integration device 300 may share in two networks, by the two networks do not communication. For example, a integration device 300 of a reading group may be connected to multiple unaffiliated hospitals. As noted herein, the integration devices of the hospitals may support sending (tunneling) of data to a reader wherever he is, but remain functionally invisible, in that a user in one site cannot request or see data of another site. Also as noted herein, in some cases, a user at one site may desire to send data to another site. Optionally, such sending is supported by integration device 300, for example, by "mounting" only that data for view by the other site. Such sending may be useful, for example, for second opinions and/or for handling work overflow. Other data, for example, patient data (e.g., for pre-fetching as described below) may be provided automatically via the network link or it may be requested using more formal methods, for example, an e-mail to a human.

### Manager tools

In an exemplary embodiment of the invention, device 300 includes a module of management tools 322, which may be used by a manager, for example, to control device 300, to control the network of which device 300 is part and/or for data mining.

In an exemplary embodiment of the invention, the manager tools are provided as Java applets which allow one or more of:
(a) Setting user permissions, assigning users to groups, password policies, and other standard user management.
(b) Setting up Network configuration (e.g., IP's, ports, Dicom, HL7, GRID specific).
(c) Setting DICOM forwarding rules, lifecycle.
(d) Monitoring rules, and life cycle execution.
(e) Manipulating various databases.
(f) Basically monitoring and configuring all of the activities listed above.

In an exemplary embodiment of the invention, a manager, for example, a reader group manager uses a dashboard like interface which indicates the status of various activities and/or includes alerts. Optionally, a dash board is used, for example, such as shown in "The Radiology Dashboard: A User's Guide to a "High-Performance" PACS", by Matthew B. Morgan, MD; Paul J. Chang, MD, in Appl Radiol. 2005;34(5):17-21, the disclosure of which is incorporated herein by reference. It is noted, that the dashboard in some embodiments of the invention include information across sites which is not provided in the above system, information about radiologist workload and/or availability and/or average rates and/or includes information on pre-fetch failures, network failures and/or other problems which interfere with timely and/or correct diagnosis.

### Security

In an exemplary embodiment of the invention, device 300 includes a security module 336, which optionally provides one or more of firewall, authentication, secure communication (e.g., over SSL or other encrypted connections), local data protection (e.g., against erasing and/or encryption for storage) and certificate management.

In an exemplary embodiment of the invention, data protection includes encryption of one or both of data and meta-data. In an exemplary embodiment of the invention, by managing the data even if not on integration device 300, module 336 can ensure that any accessible copy of data is protected and/or encrypted. For example, data may be forwarded with data protection instructions and/or data stored remotely may be retrieved, encrypted and returned.

In an exemplary embodiment of the invention, access control is provided including control according to one or more of user, group, site, e.g., certain types of studies can be blocked from viewing. Optionally, it is also possible to configure the system that a user will only be able to see studies referred or assigned to/by him.

In an exemplary embodiment of the invention, certain activities require permissions which are managed by module 336. For example, printing, deleting studies, modifying data and/or reading may require certain permissions which may be managed, for example, per user, group and/or site. As noted above, an entire site may be made invisible to some users or to all user sin a linked site and available only to a user of that invisible site who logs on to integration device 300.

### LDAP

In an exemplary embodiment of the invention, integration device 300 includes a LDAP module 338, so that all user management can be centralized. The LDAP module may manage the LDAP or serve as a client for an external LDAP.

### Exemplary usage scenario

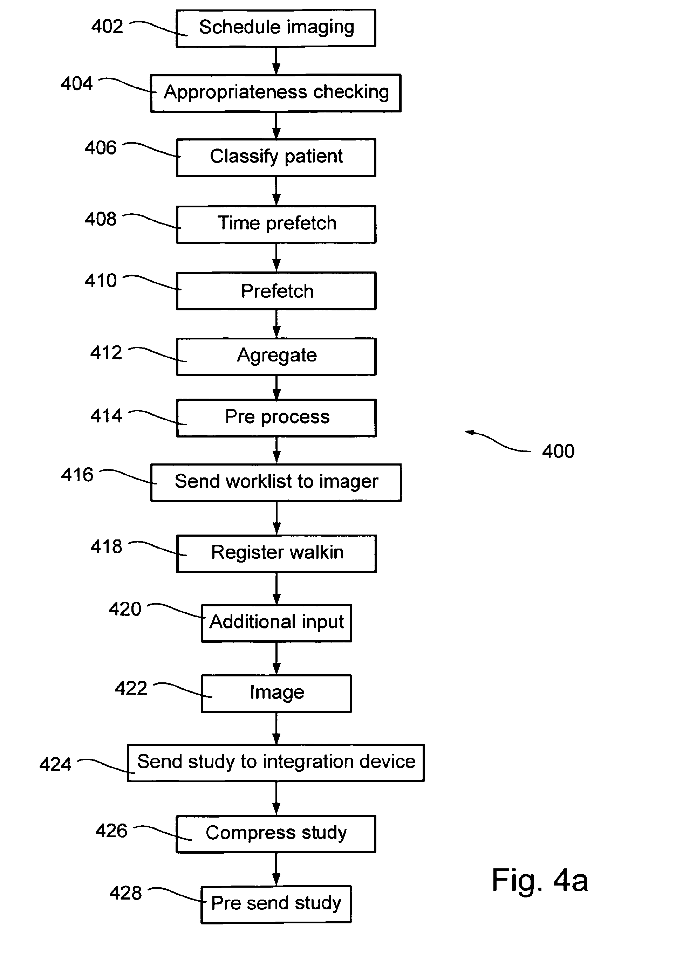

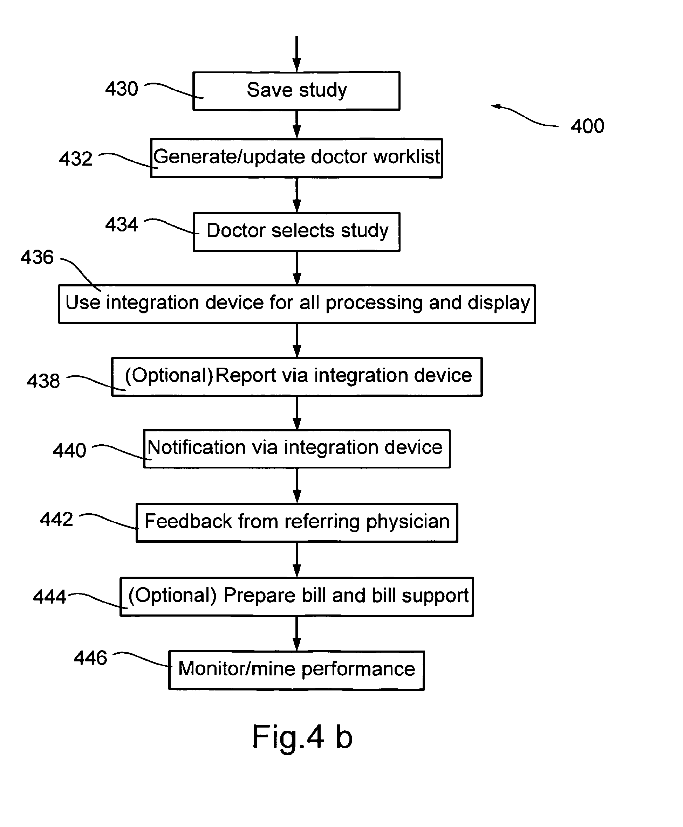

Figs. 4A and 4B are a flowchart 400 showing a process of image acquisition and diagnosis, using integration device 300, in accordance with exemplary embodiments of the invention.

### Schedule imaging (402)

At 402, an imaging session is scheduled. In an exemplary embodiment of the invention, the scheduling is via an existing RIS system and interface, which optionally sends a HL7 format order which can be received by integration device 300.

In some embodiments of the invention, integration device 300 is the RIS. In other embodiments, scheduling is via integration device 300 which emulates a user station to the RIS and which presents a user interface (e.g., a web interface) to a user. Optionally, integration device 300 requests a schedule from the RIS and base don the schedule and a user input, sends a request to set up an imaging session, to the RIS. Optionally, when a confirmation is received, this confirmation is sent to the user. Optionally or alternatively, integration device 300 retrieves the schedule periodically from the RIS and/or HIS.

In an exemplary embodiment of the invention, the process of imaging request includes an indication of a desired diagnosis, observation and/or differential diagnosis. In an exemplary embodiment of the invention, this request is in the form of a form, which is then returned filled out (as the report) to the referring physician. Optionally, such indications are used as a guide during data acquisition, pre-processing, diagnosis, report generation and reporting. For example, each such process may include different rule sand/or settings for different requests. Such rules may be stored and/or managed on integration device 300, or be on the relevant devices (e.g., imager, PACS station).

In an exemplary embodiment of the invention, the scheduling is assisted by one or more system tables which indicate which diagnosticians are available at what times. Optionally or alternatively, the referring physician indicates (or the system is aware) when he will be available to review the results and/or when a next visit is scheduled for the patient being imaged.

### Appropriateness checking (404)

In an exemplary embodiment of the invention, the appropriateness of the request is checked, for example, by comparing request to insurance coverage, indicated disease, patient history and/or physician (referring or diagnostician requested). Optionally or alternatively, the appropriateness checking is between medical centers, for example, avoiding repeating a same test at multiple centers. Optionally, such appropriateness checking is carried out after patient classification and/or after pre-fetching of additional patient data. In an exemplary embodiment of the invention, appropriateness checking includes one or more rules that flag a suspected case of medical fraud and/or a case where a reduced cost replacement may be available, and which optionally require special authorization (e.g., from referring physician and/or HMO) to overcome. For example, a rule may flag two CTs to view a hip, when a cost saving option of x-ray is suitable according to the medical indication. Optionally or alternatively, appropriateness checking includes applying one or more medical rules that flag if the accepted practice for the indication is not the requested procedure. For example, an MRI for a broken bone is typically not medically appropriate, nor is a breast biopsy in a male with a foot abscess.

### Classify patient (406)

In an exemplary embodiment of the invention, various patient matching logics are used to identify, for example, if the patient is a patient on file or not. Optionally or alternatively, matching up of suspected patients into a single patient is done manually, by presenting a query to a user.

In an exemplary embodiment of the invention, the matching is done using one or more of the following parameters:
(a) Patient ID, Assigning authority (a unique patient id issuer) - a unique ID created by the RIS;
(b) Patient First & LAST Name;
(c) Patient birth date; and
(d) Patient sex.

Optionally, if all of the above match a particular existing patient, the new patient is associated with an existing one. If not, then a new patient is created.

Optionally, there is manual intervention when the patient id, assigning authority combination is not unique, for example, if (apparently) two patients with different names get the same pid from the same assigning authority.

### Time Prefetch (408)

In an exemplary embodiment of the invention, data for a patient is not immediately prefetched, but only at a certain time before the imaging session. This may reduce load on local storage devices. Optionally, an imaging request is indicated with the type of prefetch needed and/or expected duration, which may be used for rescheduling imaging and/or diagnosis sessions, if earlier dates and/or alternate locations become available. Optionally, the request can indicate alternative imaging modalities and/or alternative readers.

### Prefetch (410)

In an exemplary embodiment of the invention, data is pre-fetched, in preparation for imaging, diagnosis of imaging and/or usage by referring physician. Optionally, the prefetch function may be used not during imaging, for example, to assist a physician or other user in collecting a complete file on a patient.

In an exemplary embodiment of the invention, pre-fetching includes determining what information to look for, for example, using a set of rules that are matched to the imaging request.

In an exemplary embodiment of the invention, a second set of rules are used to generate search locations for the useful data that is indicated by the above set of rules.

In an exemplary embodiment of the invention, a common repository ("global grid" or "local grid") is provided and a simple query, asking for all the data is performed. Optionally or alternatively, multiple integration devices can be queried and the result aggregated.

In an exemplary embodiment of the invention, data is loaded according to a predefined rule for a particular examination type. Optionally or alternatively, data is loaded from a location determined as follows. If there is only one location for the desired study, that is the source. If there is more than one location, then a score is assigned to each location, the location with the highest score "wins". In an exemplary embodiment of the invention, the function that calculates the score is base don two parameters: will the study be streamed (e.g., received lossy and/or lossless, based on the network bandwidth to the destination), and the type of media the study is located on (fast disk vs. tape for example).

Optionally, lossless and fast disk gives a better score than lossy on tape. The scores of other combinations are optionally determined by a configurable weight system (e.g., a table) that allows the function to be customized according to a customers needs, optionally including an indication of cost to retrieve data form one location as compared to another.

Alternatively, data is searched for in all the network. Optionally, the searching is facilitated by storage of meta data on the integration device 300 devices and/or at the data center, in that the searching can first identify meta data and base don the meta data retrieve the data from a location indicated or suggested by the meta data.

Optionally, the results of such a search include an availability of data. For example, data may be stored on a removable/archived storage and/or may require time until it may be sent over available bandwidth.

Data is optionally fetched in an order of priority as indicated by the rules (within a case) and/or according to schedule of imaging (e.g., between cases). For offline data, a request to retrieve the data, with an indication of needed delivery time, is made.

Examples of data which may be pre-fetched, include: previous studies, case studies, patient history, laboratory results and/or reports.

### Aggregate (412)

In an exemplary embodiment of the invention, as data arrives, the status of the imaging request is updated. For example, the status can indicate if all data arrived, if all data marked as "required" by the above set of rules arrived, if any data still is expected to arrive, if the status is known and/or if any data is missing. In some cases the studies will not be available to the radiologists until they fully arrive (e.g., as indicated by a status change). Optionally, studies which fail to arrive within a certain time frame, are flagged, for example, to a system manager or reader.

In an exemplary embodiment of the invention, data continues arriving (even after imaging) and is added to the file, to be used, for example, for diagnosis and/or by referring physician. Optionally, once aggregated, such a file is associated with a patient record and updated when more data is generated.

### Pre Process (414)

In an exemplary embodiment of the invention, pre-fetched data is prepared for later use, for example, being compressed and/or layered for low-bandwidth lines, new views being created (e.g., to match expected imaging views). Optionally, the imaging technician looks at pre-fetched data and/or request and determines (alone, with a radiologist and/or with an expert system) what imaging settings to use.

In an exemplary embodiment of the invention, the pre-fetched images are available for the radiologist as he/she diagnoses the primary case.

In an exemplary embodiment of the invention, the data is pre-processed, even if it is not clear data will be needed. Optionally, pre-processing with a large associated cost, requires user approval and/or an indication of a probable use.

Optionally or alternatively, pre-processing includes sending the aggregated data to where it may be used, for example, to an integration device at a reading group, to a reader home station and/or to a 3rd party diagnosis station where reading is expected to happen (e.g., within same hospital as imager).

In an exemplary embodiment of the invention, the aggregated data is collected into a single collection (e.g., a directory) to which a link is created and stored in a database. Optionally or alternatively, these studies are automatically/manually pushed to a user's workstation which is connected on a slow line.

### Send worklist (416)

In an exemplary embodiment of the invention, integration device 300 creates a worklist for the imaging device(s) and sends this list. In other embodiments, such a list is created by and sent by a RIS system external to integration device 300, optionally with a copy to integration device 300.

### Register walk-in (418)

In an exemplary embodiment of the invention, when a patient comes in to be imaged, this arrival is registered at a RIS system and/or at or via integration device 300. Optionally, the RIS notifies integration device 300 when such walk-in occurs. Optionally, in response to a walk-in, any pre-fetching and/or aggregation is accelerated and/or data is sent to an imaging station and/or diagnosing station.

For example, such a walk-in triggered pre-fetch may be useful for emergency cases, such as, for example, accident victims and hospital ICU patients. Optionally, a certain bandwidth is reserved for use for such short-notice pre-fetches.

### Additional input (420)

Often, an arriving patient comes with print copies of studies, laboratory results, digital (flash memory, DVD) copies of studies and/or other information (such as patient facial photograph). Optionally, patient validation uses such data, for example, fingerprints. In an exemplary embodiment of the invention, such data is scanned in when the patient arrives, for example, using means known in the art, with the process optionally being managed by integration device 300, and made part of the aggregate file (above).

In an exemplary embodiment of the invention, integration device 300 provides an interface to an existing RIS (or as RIS 318), while adding functionality, for example, showing a user photograph in a window of "additional information", which can be used to enter and/or view information managed by integration device 300 and not by the RIS. Such a feature of added functionality may be provided for other hospital information systems as well. Optionally or alternatively, existing data may be displayed in a more convenient manner.

### Image (422)

Prior to imaging, the aggregated file is optionally used to plan the best imaging sequence. For example, previous images may suggest what a particular set of imaging parameters might show or may be required to be repeated.

The patient is then imaged and new imaging data is collected and ultimately linked with the aggregated data and sent for diagnosis.

If integration device 300 acts as a RIS, it updates the local PACS (e.g., using the "modality performed procedure step" mechanism) that the imaging was carried out.

### Export study (424)

In an exemplary embodiment of the invention, the imaging device is set up to send the imaging data to integration device 300. Optionally or alternatively, the data is sent to the RIS and/or PACS, which is set up to forward a copy to integration device 300; or integration device 300 may request a coy form the PACS, for example, based on an imaging schedule integration device 300 holds and/or periodically. Optionally, integration device 300 eavesdrops on the other's communication to obtain such information. If sent directly to integration device 300, integration device 300 optionally forwards the data to the PACS and/or other defined system(s).

In an exemplary embodiment of the invention, the study data is compared with RIS data and/or various patient matching logics applied.

Optionally, if integration device 300 does not act as a RIS, then the followings is carried out:
(a) before deciding if this is an existing patient or not (e.g., as described above) the information passed from the Modality is verified using the RIS information. In general, the information coming from the RIS is always considered more reliable than the modality (if the modality obtains the information by querying the modality worklist there should not be a problem, but this is not always the case.
(b) integration device 300 locates the relevant order for the stored study, optionally by comparing a field called "accession number" which is shared on multiple hospital information systems.
(c) once the order is found, the patient information is taken from the order and optionally overrides the patient information in the study.

### Compress study (426)

Optionally, the data is preprocessed, for example, compressed by integration device 300. Optionally, the pre-processing takes into account a desired diagnosis (e.g., allowed quality reduction and/or artifact types) and/or a target location (e.g., bandwidth, layer preparation).

### Pre-send study (428)

Optionally, even if an exact reading location is not known, study data and/or aggregation data may be sent to an expected reading location and/or data center. Optionally, this is done using an automatic rule such as "all studies of type X are copied to a destination Y or pushed to a certain user Z. Optionally, the data is sent with a dating indication, indicating when to erase the data if it is not used. Optionally, the network of integration devices 300 manages the location of the "source" data and may also monitor the locations data was sent to. Optionally or alternatively, if data is used at one location, a signal to erase the data may be sent by integration device 300 to a storage device at a different location to which data was sent, but will not be used.

### Save study (430)

Before, during or after sending out of the study, a backup copy of the study is optionally made. Optionally, this saving is part of a starting of the lifecycle of the study, which can include, for example, periodic backups, different levels of availability and/or deletion.

### Generate/update worklist (432)

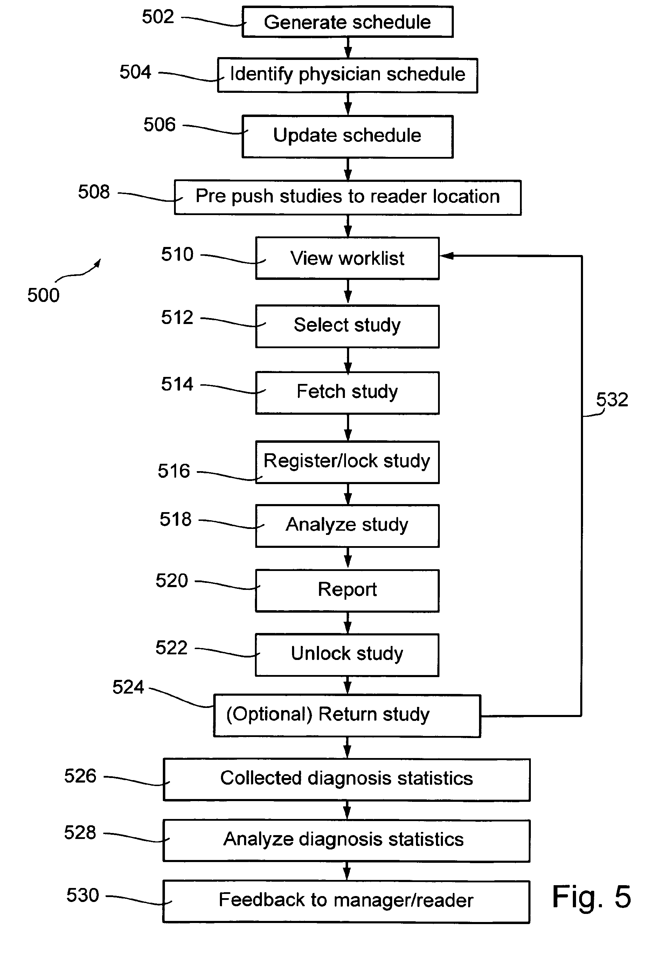

As noted above, the reader worklist may be updated continuously, for example, as new imaging requests are made. Optionally, the worklist is updated when a new study is actually acquired (e.g., whether by imaging or otherwise, such as by electronic delivery for "second opinions"). Additional details regarding some exemplary embodiments of scheduling are provided with respect to Fig. 5, below.

### Doctor select study (434)

A doctor views his worklist and the various priorities and deadlines and selects one or more studies to view. In a setting with reduced bandwidth, a plurality of studies may be more useful to select as they may be streamed during diagnosis of a fist study.

### Diagnose using integration device (436)

A particular feature of some embodiments of the invention is that substantially all tools needed for diagnosis are expected to be found at a same workstation, for example, at least 70%, 80%, 905, 95% or intermediate percentages of time may be spent diagnosing at the same station. Optionally, the tools are provided from integration device 300 to a client station.

In an exemplary embodiment of the invention, the data used for diagnosis includes the study and the aggregated data, one or both of which may be provided, for example, on the fly, previously stored or provided on the fly from another integration device 300 where the data is stored.

In an exemplary embodiment of the invention, a doctor/reader may ask for additional studies and/or data for the study and be informed as to the time it will take to receive the studies. Optionally, the estimation is based on a combination of bandwidth and media type. In an exemplary embodiment of the invention, a notification is sent to doctor, for example, via SMS, instant messenger, screen pop-up and/or via the study appearing on the physician list. Optionally, such a notification is also provided to a manager (e.g., to a dashboard thereof). when the requested data arrives, or its arrival is imminent or its arrival is in doubt or greatly delayed.

In an exemplary embodiment of the invention, the display profile used to display the study depends on one or both of the physician and the indication. Optionally or alternatively, the data is displayed in a manner compatible with previous studies.

In some embodiments of the invention the display protocol system allows a user to set up a particular display scheme for a particular type of examination. Once this is done, all future studies matching the criteria will be displayed in a similar way. For example "Display all CT head cases with an MPR reconstruction on the right screen, and VOLR on the left. Optionally, the display protocol includes a preprocessing protocol (e.g., generating the MPR, or marking or artifacts) which is optionally performed on integration device 300 or on the client.

In an exemplary embodiment of the invention, data is provided in an order according to the desired diagnosis. Optionally or alternatively, tools suggested to the physician are according to the desired diagnosis.

### Report via integration device (438)

In an exemplary embodiment of the invention, reporting is performed using integration device 300. Optionally or alternatively, the diagnosis process is structured in a manner that supports a report structure. Optionally, a report template is used which matches the desired answers.

In an exemplary embodiment of the invention, actual reporting is image based, with a physician indicating images and marking up the images as part of the report. Optionally or alternatively, the report is voice based, with physician dictating report to integration device 300. In an exemplary embodiment of the invention, the desired diagnosis and/or other clinical information is sued to assist in interpreting the physician's voice. For example "femur" is not a word expected in mammography analysis. Optionally or alternatively, the reporting is via integration device 300 which passes the raw report data (e.g., images, voice) to an existing, external, report generating system. Optionally, any draft report is sent via integration device 300 to the diagnosing physician. Optionally, when such a draft arrives, the study to which it refers is uploaded as well (or otherwise guaranteed to be available). Optionally, this is true also if the draft report is reviewed at a different location.

In an exemplary embodiment of the invention, integration device 300 sends the report to the RIS and/or PACS. Optionally or alternatively, if the report is created on the RIS, when ready, it is sent to integration device 300, where it is optionally stored and/or sent for storage.

### Notification via integration device (440)

In an exemplary embodiment of the invention, integration device 300 generates a notification to the referring physician, for example, an urgent notification (e.g., via SMS, instant messaging, fax, voice message, or other options) if the report indicates an urgent notification is appropriate. Optionally or alternatively, integration device 300 generates an e-mail or paper report to the referring physician. O the e-mail includes links to the images. Optionally, integration device 300 is the address of these links, which it translates into links to the actual storage. Thus, integration device 300 can provide relatively stable links to data which may migrate according to storage needs and/or availability.

Optionally, the report is created on the RIS but still published by integration device 300 with the RIS being programmed with integration device 300 as addressee for all reports.

In some cases, the report is prepared on one integration device 300 and forwarded to another integration device 300 which then forwards the report to a RIS or other system.

In a particular example, a user connects with a client to one integration device and loads a study from a worklist in the data center. The study is fetched from a second integration device. The user creates the report on the client and saves it to the second integration device, by tunneling the communication through the first integration device and the datacenter. From the second integration device the report is sent to the RIS at the site of the second integration device.

### Feedback via integration device (442)

In an exemplary embodiment of the invention, integration device 300 is used to provide feedback to other users of the system. In one example, of feedback, a referring physician can post note and/or questions to a report he receives, via the report (e.g., the report including a clickable button or contact information for questions) and these questions are forwarded by the system to the original reader and/or to a second opinion reader. Optionally, the quality and time to receive answers are monitored by integration device 300. In another example, a surgeon, performing surgery (or other therapist/therapy) being guided by the study, may indicate of the study and/or diagnosis were correct and/or useful and/or provide suggestions for improvement. Optionally, the reader already includes options in the report, which options may be indicated by the other users, to provide more exact feedback to the reader (or image acquisition technician) as to what would have made/did make the study useful.

### Prepare bill and bill support (444)

In an exemplary embodiment of the invention, in anticipation of possible payers complains about a bill, the bill is prepared in conjunction with a file of support for the bill. Optionally, the RIS generates an output of what was actually done (e.g., in response to technician and/or integration device 300 request) and then this information reported to billing system (external system). Optionally, integration device 300 prepares a bill report which can be used by a bill checker and which may include, for example, links to the imaging report and/or patient data. Storage can be, for example, local, optionally with a link (e.g., permanent) provided to the billing system. In an exemplary embodiment of the invention, links are managed via archive manager 302, which translates incoming "links", using a table or database into actual storage locations. As noted above, the actual storage locations may change. Optionally, some links are defined to be deleted or otherwise not supported after a time or as part of a garbage collection process.

### Monitor/mine performance (446)

In an exemplary embodiment of the invention, the data collected in integration device 300 is analyzed for one or both of monitoring performance (e.g., of readers) and mining the data for various trends (e.g., usefulness of various procedures). It should be noted, that the above integration of data and process control may make information not previously available for analysis and/or monitoring, available. Additional details are provided below.

### Data flow control

A feature of some embodiments of the invention, is that the network of integration devices 300 can be used to reroute information according to need. For example, considering a network including hospitals, remote users and data centers, all interconnected by integration devices 300 and by multiple connection types (e.g., ADSL, WAN, LAN, point-to-point link). Furthermore, rerouting can be scheduled to take advantage of links that are temporarily free instead of waiting for over-loaded links.

In one example, a reader in one hospital can obtain data from a second hospital via a integration device 300 of a third hospital.

In an exemplary embodiment of the invention, each integration device 300 is responsible to configure the connection to other integration devices it communicates with. Optionally or alternatively, some or all of the integration device will forward (e.g., data and/or requests) to a central node, such as at a data ceneter, which will distribute the messages.

In another example, data that is temporarily stored at a integration device 300 is provided instead of original data if the data is not immediately available from the storage location of the original data. This substitute data may be appropriately marked to the user, especially if it has a checksum not matching that stored as meta data or has an older date.

In another example, a home user is presented with a worklist according to the instant availability of data links to the data.

### Failure management

In an exemplary embodiment of the invention, integration device 300 is used as part of a failure management system.

In an exemplary embodiment of the invention, integration device 300 serves as a backup of data, as most or all recent data in the HIS, RIS and PACS pass through integration device 300 and are optionally backed up or stored thereby.

In an exemplary embodiment of the invention, integration device 300 can provide RIS, PACS and or other image processing services instead of existing components. Thus, in case of failure of an existing RIS system, for example, users can use the RIS system provided by integration device 300. Optionally, such a system also

In an exemplary embodiment of the invention, failure resistance is provided by integration device 300 serving as a manager of network connections.

In an exemplary embodiment of the invention, integration device 300 acts as a backup server which attends to backing up data and thereby supporting recovery from failure.

In an exemplary embodiment of the invention, integration device 300 is inherently failure resistance by using a standardized architecture which allows relative quick replacement by an alternative standardized element. In an exemplary embodiment of the invention, in a site where there are multiple integration devices 300, if one integration device 300 fails, the other integration device 300 can take over, optionally having stored (or having access to) a copy of the relevant configuration data and/or medical data or meta data.

In an exemplary embodiment of the invention, integration device 300 is used in detecting equipment failure, for example, by detecting lack of response to messages or increased response time.

In an exemplary embodiment of the invention, integration device 300 is configured with one or more other integration devices 300 in a site or off-site, to serve as hot-backups for each other. Optionally, all information is mirrored between the devices. Optionally or alternatively, the information is not mirrored in that ongoing processes may crash if a box fails, but no data is lost, or at least not more than a last and on-going diagnosis.

Following are some examples of managing failure using integration device 300, in accordance with exemplary embodiments of the invention.

In an example of local PACS failure:
(a) Local modalities start sending new studies to integration device 300. Optionally integration device 300 changes their set ups using a predefined configuration file, or a user is given specific instructions.
(b) Local users read new studies of integration device 300 either with the client of integration device 300 or using 3rd party workstations reading off integration device 300 using DICOM.
(c) Once the local PACS is back online, integration device 300 sends accumulated studies, reports to the local PACS.
(d) If data on local PACS is permanently lost, integration device 300, as part of the lifecycle mechanism can restore at least some local PACS data using the local cache of integration device 300 and/or using data belonging to the specific site which is stored on a central or local disaster recovery site.

In an example of local RIS Failure:
(a) All RIS scheduling activity is done through the scheduling module of integration device 300.
(b) All modalities start working with the modality worklist from integration device 300.
(c) All reports are accumulated in integration device 300.
(d) Once the RIS is back online, all accumulated data is sent to the RIS.

In an example of hardware failure of integration device 300:
(a) Optionally, two integration devices are connected together in a high availability cluster.
(b) A heartbeat mechanism is used to ensure that both integration devices are online.
(c) Once hardware failure occurs, the remaining integration device 300 takes the function of the failed one.
(d) A virtual IP mechanism makes sure that this change over is transparent to the other components/systems on site and off-site.

### Interoperatability, scheduling and remote access

A particular feature of some embodiments of the invention is providing healthcare networks with the ability to synchronize multiple and disparate RIS/PACS into a single platform without having to replace existing systems.

Fig. 5 is a flowchart of a method 500 of work planning and image diagnosis across hospital networks, for example from home, in accordance with an exemplary embodiment of the invention. In some embodiments, these methods are used for s a single platform/site implementation.

Acts 502-508 relate to creating and updating a cross-platform schedule. Acts 510-524 relate to the diagnosis and acts 526-530 relate to monitoring such work.

### Scheduling

At 502, a schedule for the readers/radiologists is generated. Such a schedule may be based on one or more of the following, taking note that some of the information is available due to the integration and/or other features of integration device 300 as described hereinabove:
(a) Reader abilities, for example, what types of studies can the reader read and/or typical reading times.
(b) Reader work schedule.
(c) Reader non-diagnosis work schedule.
(d) Payer preference, e.g., for reader or cost.
(e) Reader cost (possible with extra cost for "rush" jobs) or other cost logic as negotiated with work provider (hospital).
(f) Reader's need for supervision and/or availability of such supervision.
(g) Resources needed (and availability) to provide data to reader at expected reader location.
(h) Expected diagnosis tools at expected reader location.
(i) Referring physician preference for reader(s).
(j) Needed/desired diagnosis report schedule.
(k) Reader preference, for example, for certain types of studies and/or runs of study types and/or rate of change of work schedule.
(l) Training schedule for a reader, which requires, for example, a minimum number of diagnoses of various types.
(m) Labor allocation to different hospitals.

In some cases, the schedule is prepared ahead of time, for example, when the imaging study is ordered. In an exemplary embodiment of the invention, the schedule is updated (506), for example, in response to change sin workload and/or in response to actual reader availability (504), which may be monitored by a hospital attendance system or by a reader group schedule system which interfaces with integration device 300. Optionally or alternatively, the schedule is updated according to a tracking of actual diagnosis progress by the readers.

It is a particular feature of some embodiments of the invention, that on-line availability of data re studies needing diagnosis and availability of readers is used to continuously optimize reading schedule.

Optionally, a reader group manager can modify such a schedule, for example, using management tools. In an exemplary embodiment of the invention, a reader group manager can physically allocate the study to a different physician. Optionally or alternatively, the radiologist is provided with the ability to reject an assigned study, optionally triggering the sending of an alert to the reading manager. Optionally or alternatively, any study which is ignored for a certain (optionally study-dependent) amount of time, causes an alert to be sent.

At 508 data needed for reading is optionally pushed to reader location. Optionally, the data is pushed to several locations according to an instant probable reader and/or costs of such pushing. In a particular example, home reading, a plurality of cases, for example ten, are pushed to a home location. In one example, a reader indicates he is changing location and a set of relevant cases are forwarded to the new location. Optionally, the reader also indicates expected arrival time at the new location.

In an exemplary embodiment of the invention, a user indicated an expected schedule using an interface that shows days and hours, or just days with an option of entering hours. Optionally or alternatively, the data is entered by a secretary/administrator.

In an exemplary embodiment of the invention, the system (integration device 300, via client software on the user's workstations and/or via a monitoring of logging-in activities) will detect which users are currently online and their availability (e.g., network bandwidth) and/or how many unread studies are currently assigned to them (which may be another factor on where to assign a new study)

In an exemplary embodiment of the invention, the scheduling (and data routing) take into account that some studies may require multiple stops. For example, a study may require that a second person review the report and/or sign on the report. Optionally or alternatively, some readers may be defined as being available for consult on complicated cases and/or for monitoring a different reader. The schedule optionally includes scheduling the availability of the multiple readers in a useful and timely order.

It should be noted, that in general, the schedule includes tasks from multiple hospitals, data vendors and report expectations.

### Diagnosis

An exemplary such process has been briefly described with respect to Figs. 4A and 4B as well.

At 510, a reader reviews his worklist. As noted above, the worklist optionally includes tasks from multiple hospitals and may include indications such as urgency and/or special requests

At 512, the reader selects a study, for example, the first one (which may be automatically selected and displayed by the system), or based on convenience. Optionally, if the selection causes a more important study to be in danger of not being done on time (e.g., time left in workday less than time to do study and/or study needed fast), an alert to the user and/or his manager, is generated.

At 514, the study is fetched, if not already fetched. Optionally, at least a check to see if any updated data or partial diagnoses are ready is made.

At 516, the study is locked for changes by others. Optionally or alternatively, the study is registered as having its definitive version located at the integration device 300 where diagnosis is being carried out form.

At 518, the reader performs the diagnosis. Optionally, one or more tools for diagnosis are provided based on information with respect to the desired result. Optionally or alternatively, tool parameters are automatically set, for example, windowing parameters for an image processing tool may be automatically set based on diagnostic information for the RIS and/or base don imaging parameters. Optionally or alternatively, non-DICOM objects provided with eth study may be launched, for example, for showing documents or audio files. Optionally, such launching is using well known techniques of identifying an application to use based on file type.

In addition to DICOM images, metadata and non-DICOM objects are also available for the diagnosis process and non-diagnosis processes, for example, laboratory reports, and movies, for example of a laparoscopic procedure, by the mediation of integration device 300.

At 520, a report is generated, for example, using integration device 300 or using alternative reporting systems. In an exemplary embodiment of the invention, the reporting is performed using a format as accepted by the study provider. In an exemplary embodiment of the invention, integration device 300 provides a template and/or formatting and/or link to reporting system for the report according to the study originator and/or other details in the study request.

At 522, the study is unlocked and may be transported to a new location (524, optional).

At 532, this studying process may be repeated until the worklist is empty and/or reader leaves. If there is unfinished work, such work may be rescheduled by integration device 300. Optionally, a reader can indicate that some urgent work cannot be done on time and it is immediately rescheduled or reported to a workflow manager by integration device 300.

As may be appreciated, the above architecture can allow radiologists serving multiple healthcare facilities to initiate virtual reading and reporting across large regional or national geographies. Optionally or alternatively, a hospital may allow multiple non-associated reading groups to work on a same global worklist of diagnoses to do for the hospital. In one example, one group does the diagnosis and the second group checks the diagnosis.

### Monitoring

As noted above, a particular feature of some embodiments is the on-line availability of information that makes it possible to monitor, optionally in real time the output of the diagnosis process and identify potential problems, while allowing a more correct distribution of effort than in the art, especially across disparate clients.

In an exemplary embodiment of the invention, various diagnosis statistics are collected (e.g., and displayed in real time).

In an exemplary embodiment of the invention, a dashboard type display is used. Optionally, the data shown is the data as collected by the database of integration device 300 such data may be substantially real-time, for example, be up-to-date at a resolution of hours, minutes or even less than a minute.

In an exemplary embodiment of the invention, one or more of the following items of data are collected and/or displayed:
(a) Studies per hour (e.g., stored, read, reported);
(b) Number of concurrent users (radiologists and/or referring);
(c) Number of concurrent modalities in action;
(d) Average time for study turnaround;
(e) current state of the system, e.g., one or more of Free CPU, Memory, Database space and Free space on any of the TIERS;
(f) Number of studies awaiting backup;
(g) Number of studies awaiting metadata sync with other obx;
(h) Number of metadata received from other integration device;
(i) Per reader, the queue of studies waiting;
(j) reader overload;
(k) reader diagnosis speed;
(l) reader mistake rate; and/or
(m) reader availability.

At 528, the statistics are optionally analyzed, for example, using rules, into information, for example to identify possible problems and/or opportunities. For example, it is possible to calculate how much output a specific radiologist provides and then promote/fire/chastise him. In another example, it is possible to see if any particular modality is offline (e.g., broken) more often than others and possibly replace/maintain it. In another example, it is possible to preempt problems before they occur, by identifying pre-trouble indicators, like missing radiologist being identified before a real backload appears.

At 530, feedback is provided to a manager and/or to a reader. In the case of a manger, the information is optionally presented in the form of a dashboard of vital statistics and optional with a list of reader and/or hospitals and/or cases that are problematic and/or a list of readers that are underutilized.

In the cases of a reader, the information is optionally provided to show typical reading time and whether or not to expect to complete the worklist.

In an exemplary embodiment of the invention, the provision of an integrated system is used to allow consulting on a case between readers that are in different locations. For example, a first reader can select a logged in reader to consult with and data for consulting is forwarded to the other reader, be the other reader at a different location and currently diagnosing data provided by a different system. Consulting may wait for all data to arrive or may occur as soon as enough data arrived. Optionally, a consultant is selected according to quality (e.g., bandwidth, availability) of data link and/or other site and/or person specific data managed by integration device 300. Then various cooperation methods known in the art of web sharing may be applied, for example, shared cursor and reflexive annotations. Optionally, a voice channel is provided by integration device 300 as well. Optionally, this process is used for monitoring by a supervisor or for signing of reports while a student watches by. Optionally or alternatively, a chat is opened for cooperation. Optionally or alternatively, instant messenger applications are used. Optionally or alternatively, "sametime" integration is used. Optionally, shared reporting is supported, for example, one reader viewing a report as it is entered and/or optionally being able to annotate it. It should be appreciated that such features can leverage known groupware technologies to the field of radiology, at least in part by integrating across disparate platforms and locations.

### Configuration

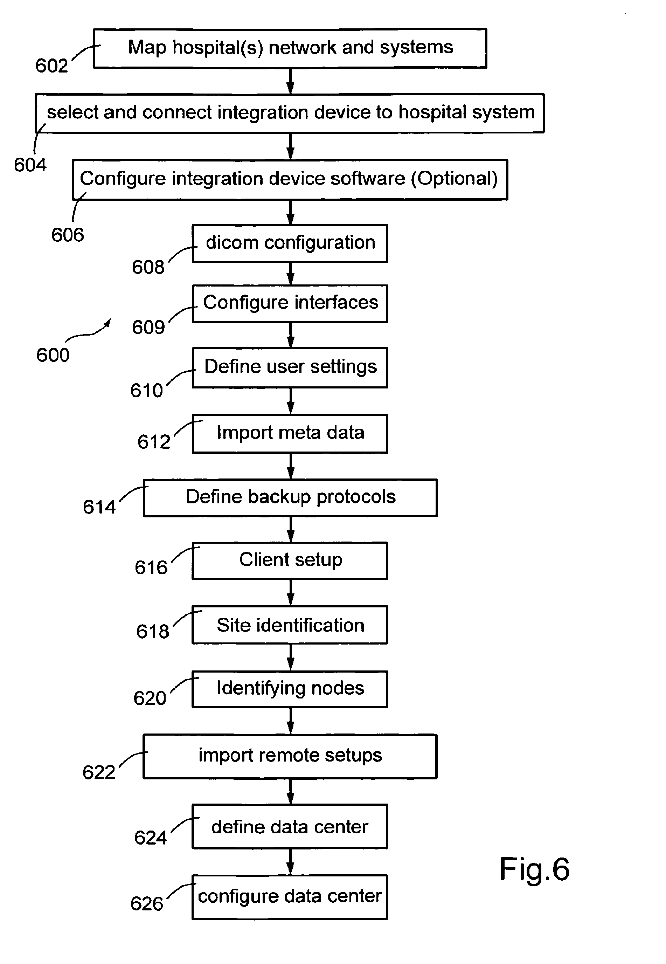

Fig. 6 is a flowchart of a process 600 of configuring one or more of integration device 300, in accordance with an exemplary embodiment of the invention.

In a typical installation, integration device 300 is provided as a retrofit to an existing complex and often outdated installation. In some cases, device 300 is installed and then gradually takes over tasks of existing installations. For example, a integration device 300 may be provided that takes over the task of a RIS for one department and then later, for additional departments. Optionally or alternatively, other hospital systems are integrated into integration device 300, such as a HIS, although being non-radiological, it may be less common.

In an example of taking over a PACS system:
(a) All system components are configured to work with integration device 300 instead of the legacy PACS.
(b) Integration device 300 performs a metadata takeover of the legacy PACS.
(c) At this stage integration device 300 has a full list of the studies, but every load request is forwaded to the legacy pacs.
(d) Gradually integration device 300 moves the actual data from the legacy PACS to the storage managed by integration device 300.
(e) The legacy PACS is removed from the site.

In some cases, integration device 300 is provided as part of setting up a new installation, for example, a new data center or new reading group site.

In some cases, an existing device is upgraded to be a integration device 300. Optionally, such updating is by adding hardware and/or software.

The following description uses a hospital retrofit as a non-limiting example.

### Map site (602)

At 602, the desired site is optionally mapped, for example, with respect to number of studies (e.g., procedures/year index), number of users on slow lines, total number of users, average concurrent and/or maximum number of users, future plans, identification of systems and/or power and cooling supplies.

### Select & connect (604)

In an exemplary embodiment of the invention, based on the map and/or a desire for future growth, a specific configuration (e.g., CPU, memory, disk) for integration device 300 (or several such devices) is selected (e.g., including processing power, storage, bandwidths and/or memory. Optionally, more than one box are selected, for example, if the total load is above a threshold or if a higher availability is desired.

An integration device 300 is selected according to the desired configuration and physically installed in the hospital (or other site), by connection to LAN, power, cooling and/or storage networks (such as SAN, NAS, CAS, storage switch, LAN).

### Software configuration (606)

At 606, a software configuration is optionally carried out. Such configuration may include, for example, IP settings, installation of non-standard emulators and interfaces and/or deletion of unneeded interfaces and/or drivers. In an exemplary embodiment of the invention, any needed drivers are provided by with integration device 300 so that configuration is minimized.

Optionally, the configuration includes setting up users on the RIS systems, etc. for the RIS (and other) interfaces of integration device 300 to use in communicating.

Exemplary site-specific customizations include:
(a) Redundancy: some customers require different redundancy policies - for example, on some sites two copies of the data are maintained for disaster recovery.
(b) Life cycle: different countries enact different rules on how long data must be kept. This might be also different for different examination types (i.e. Mammography vs. Chest CR).
(c) Site specific hanging protocols.
(d) Site specific common folders.
(e) Site specific mapping of RIS/HL7 fields to the studies database.

However, it should be noted that many such customizations require only setup and do not require complex programming.

### DICOM configuration (608) and interface configuration (609)

In an exemplary embodiment of the invention, integration device 300 is configured with DICOM sources, for example, scanners, PACS system, data centers and other integration devices.

In an exemplary embodiment of the invention, the various hospital devices are configured (e.g., imagers, scanners) to send data to integration device 300.

In an exemplary embodiment of the invention, various hospital systems, such as RIS, HIS and PACS are configured with regard to their interface with integration device 300, for example, being configured to send messages to integration device 300. In an exemplary embodiment of the invention, PACS is configured to send data to integration device 300. Optionally or alternatively, RIS is configured to report to integration device 300 various items, such as admissions, orders and reports. Optionally or alternatively, the reporting system is configured to send reports to integration device 300.

In some cases, it may be difficult or impossible to externalize data.

For example, if it is not possible to setup the local PACS to forward incoming studies to integration device 300, one option is to pull the information of the PACS via queries issued in DICOM to the local PACS on a timely basis. Another option is to change the workflow in the site in such a way that the modalities will send data to integration device 300 which will then forward to the local PACS.

Similarly, with respect to the local RIS, for example, if integration device 300 does the reporting, the RIS is not required to forward such information to integration device 300.

Another interface configuration is that of the HL7 system, for example, for session scheduling as described above.

### Define user settings (610)

In an exemplary embodiment of the invention, various user settings are provided, for example, names of users, permissions and/or user groups. Optionally, an application specialist sits with some or all users to fine tune hanging protocols, display settings (e.g., colors, annotations), display protocols and folders.

In an exemplary embodiment of the invention, existing user data is imported, for example, form a RIS or HIS.

In an exemplary embodiment of the invention, various security settings (e.g., access control) are provided.

### Import meta data (612)

In an exemplary embodiment of the invention, meta data stored in local hospital systems (e.g., PACS, RIS, HIS) is imported. In one example, configuration of a hospital site with integration device 300 takes several (e.g., 1-3) days, while importing meta data from a legacy system can proceed at 5000 procedures/day. Optionally, the import starts with recent studies.

### Define backup protocols (614)

Optionally, one or more data and process backup protocols are selected and/or defined. Optionally, the protocols are defined based on the specific configuration and/or load in the hospital and/or type of hardware used for storage. The protocol may also depend on other integration devices 300 connected in the network.

### Client setup (616)

In an exemplary embodiment of the invention, various client systems are configured, for example, by setting up connection and/or software for interfacing with integration device 300. Optionally, such software is installed on the fly at a first time connection with integration device 300.

Optionally or alternatively, specific integration components are installed. For example, an identifying unit may be installed on a third-party reporting system or RIS system client device. Optionally or alternatively, an integration component that allows data transfer between a RIS (or other) client and integration device 300 are provided.

### Site identification (618)

In an exemplary embodiment of the invention, the site identification is programmed into integration device 300. Optionally, the site identifications is provided to a roster of Integration devices.

### Identify nodes (620)

In case of a networked installation, integration device 300 optionally discovers or is notified of the identification of other integration device 300 to which it is to connect. Optionally, the notification is via a roster, or by manual entry.

### Import remote setups (622)

In an exemplary embodiment of the invention, meta data is imported, for example, in the form of an identification of a remote hospital setup (e.g., system/vendor definitions, data organization) and/or security settings. Optionally, such importing is from a data center. Optionally, such importing allows a single integration device 300 to operate with both a local and a remote site, for example, as a backup or for being physically transported to a site where the device is needed.

In an exemplary embodiment of the invention, security settings include settings that allow remote data to be passed through a integration device 300, but not locally read, except by a reading group. This is an example of a setup where the degree of sharing between sites is less than complete, however, the benefits of data availability for a reader may still be utilized, while integration device 300 at each hospital ensures that secrecy is preserved. This may also be considered an example of tunneling form a reader at one hospital to another hospital, however, it is noted that data for the reader may be locally stored in a integration device 300 even if not available to other local hospital systems.

### Define data center (624)

Optionally, one of the integration devices is defined as a data center. Optionally or alternatively, a regional data center is defined between the main datacenter and the sites. Optionally or alternatively, one integration device can serve as both a datacenter and a local integration device. Optionally or alternatively, the division of labor between different integration devices 300, in a same or different sites, is defined, for example, storage, default user host and/or RIS. Typically, if a site has a integration device 300, that integration device 300 will serve as a local integration device, however, that is not required. Optionally or alternatively, what is defined is the sharing of data and/or location of storage of critical data, such as configuration data.

### Configure data center (626)

In an exemplary embodiment of the invention, a data center is configured by importing meta data from other integration device 300 in the network. Optionally or alternatively, such data is shared as a distributed database configuration, where each item of data (e.g., meta data or patient data and/or image data) is optionally available from more than one source, for providing robustness.

While the above configuration process may be manual, in an exemplary embodiment of the invention, the process is partially or completely automated. In an exemplary embodiment of the invention, when a integration device 300 is connected to a system it self discovers the other integration devices 300 and the hospital systems and negotiates an appropriate set up. In an exemplary embodiment of the invention, such negotiation includes suggesting to a user how the new UI should be implemented into the hospital, for example, based on number of users and number of different systems in use.

In an exemplary embodiment of the invention, during the installation of an integration device, a URL of a datacenter (or other service location) will be entered. The integration device will then connect to the URL and configure itself (e.g., name, ip, DICOM, ports, synchronization rate, using data from the URL.

### Exemplary home diagnosis example

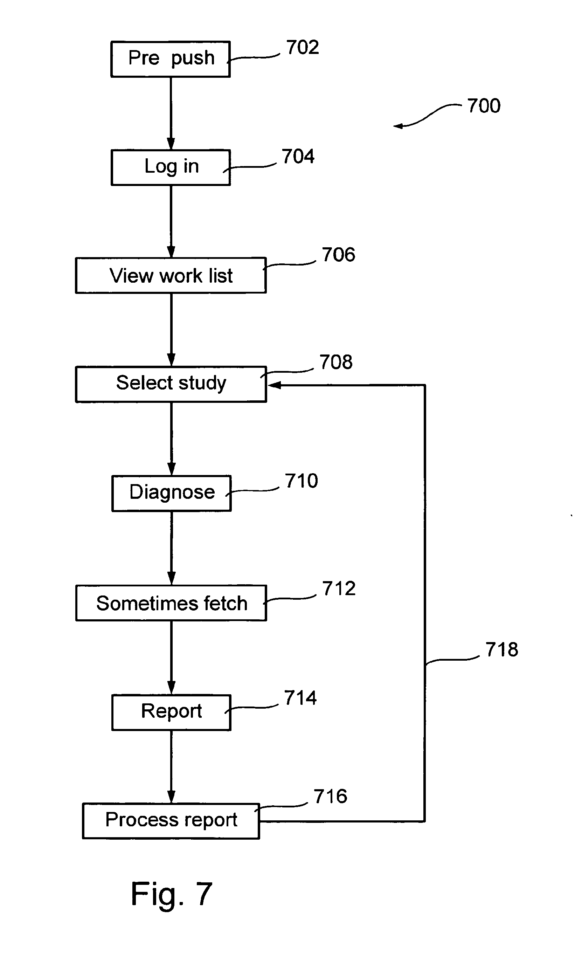

Fig. 7 is a flowchart of a method 700 of home diagnosis, in accordance with exemplary embodiments of the invention and generally following the above description.

At 702, data is pre-pushed to a home computer. Optionally, a client software is provided on the home computer and which is configured to receive and locally store such data

At 704, a user logs into a integration device 300 from his home computer, or the home computer is configured as a integration device 300.

At 706, the user views his worklist, as described above.

At 708, the user selects a study and diagnoses it (710).

In some cases, data is continuously fetched during the diagnosis, for example, related studies, layers not previously fetched and/or non-DICOM objects.

At 714 a report is prepared, optionally using a local software or a link to a remote reporting software.

At 716, the report is processed by integration device 300, for example being forwarded to integration device 300 by the reporting system. O the report is shown to the user for confirmation.

At 718, the process is repeated until work is completed or otherwise stopped.

### Exemplary integration utilization

Following are several additional examples of utilization of the integration possibly provided by integration device 300 to enhance radiology.

In a first example, a closed cycle referral-reporting system is used in which a report is designed for and anticipated by a request for information by a referring physician. By linking the structure and content of the report and allowing the actors to communicate directly, an improvement in meeting of needs and/or a reduction of misunderstanding, may occur. Such a closed-cycle may also encourage physicians to user readers they do not know personally, as a risk of misunderstanding and/or stonewalling is reduced.

In another example, a payer (e.g., hospital, HMO) has better control over costs, as it can track and request specific treatments for its needs (e.g., quality of diagnosis, number of readers, identify of readers, time scale).

In another example, a reader can have work fed to him in a more uniform manner and allowing a single interface to be used for all his needs. Optionally, by allowing the reader to access data from remote location in a seamless manner, multi-site reading while correct allocation of resources is easier to achieve.

In another example, diagnosis can be enhanced, for example, with data being pre-processed according to need (e.g., a reformatting of CT data to lie parallel to a spine, for certain spine imaging procedures), which need is imported from a RIS or otherwise available in integration device 300. In another example, data is streamed to a remote user according to the required diagnosis. For example, an image may be segmented and segments/data related to the diagnosis (e.g., bones and surrounding tissue in skeletal study) sent first and/or at a better resolution. In another example, reporting is enhanced, by suggesting images for a report, base don them including the organ of interest (e.g., a femur).

In an exemplary embodiment of the invention, a user which is connected over a slow network connection can have the study pushed in a lossless format to his local hard disk. This can be done while the user is not working (for example, at night when the user is sleeping) - once the user starts working, all the information is already available as if he is working on the local LAN.

### Maintenance

The above described architecture has various potential advantages which may be utilized in accordance with some embodiments of the invention:
(a) There is only one device to configure or reconfigure. This may simplify maintenance procedures.
(b) In a site with multiple devices, one device may take over the functions of another one, for example, for scheduled maintenance or in case of a failure. Optionally or alternatively, a device form one site may be taken to another at short notice.
(c) If the devices are manufactured to be modular, one device can be a source of immediate replacement components for another. Also, the stock of needed spare parts may be reduced considerably.
(d) Software configurations and problems are greatly simplified if there are only a small number (or one) of different software installations.
(e) Various tools, such as image processing tools, are shared across configurations and are optionally updated from remote.
(f) The full solution is managed by a single vendor, and there is a single party responsible for everything. This may also reduce integration and implementation problems.

### Specific exemplary implementation details

In an exemplary embodiment of the invention, integration device 300 is implemented as a single box. Optionally, the box is of a modular deign, for example, a blade architecture, allowing memory, storage and/or processing power to be added as desired, optionally while operating.

In an exemplary embodiment of the invention, each integration device 300 includes a database manager, for example, an Oracle database.

In an example of a medium site with 200,000 procedures/year the following configuration may be used: windows 2003 server with a quad core Pentium system, 4-8GB and 2TB disk space (e.g., 1 TB for database and OS and 1 TB for a 2 month image data cache).

In an alternative embodiment of the invention, integration device 300 is provided as a plurality of boxes, connected together, for example, by a LAN or a fast bus. In an exemplary embodiment of the invention, the multiple boxes are connected on a single LAN and are managed by an IP based load balancing component. Optionally, to the external world all the boxes look like a single entity with a single ip, Dicom AE, and HL7 interface. Optionally, this structure is internally managed and in case of failure the system optionally automatically directs all incoming communication to an available box. In an alternative configuration, each box is allocated a different function (RIS, PACS management), and serve as backups for one another (e.g., by each having a copy of a hot-synchronized database). It is noted, however, that while some of the above functionalities can be provided by such a cluster, some of the functionalities, such as a most simple installation, utilize the configuration in a single physical housing.

In an exemplary embodiment of the invention, the software used is based on, and optionally may be provided as additional modules or an upgrade to, a software used for the product "Carestream PACS", available from Carestream Health, Inc, Rochester, NY, USA.

In an exemplary embodiment of the invention, additional processing power is provided at a site by adding integration devices. In general, such scaling is linear as the processing includes many requests and the requests can be divided between devices. Optionally, one of the devices is in charge of distributing the requests, for example, using load balancing methods known in the art. In an exemplary embodiment of the invention, for example, if each device has a hot-synchronized copy of the system (meta) database, one device can be upgraded (e.g., hardware and/or software) while the other device is handling request, without interfering with hospital processes. As noted above, possibly ongoing studies will be damaged or will need to be redone (or will be manually terminated).

### Data mining

In accordance with an exemplary embodiment of the invention, it is noted that the medical process data provided by integration device 300 is not previously available. Such data can be useful to several actors, for example, a reading group, a payer and an imaging provider, as well as for strategic design purposes (e.g., future hospital and healthcare design.

In general, the above architecture enables the planning and monitoring of every productivity element and thereby provides an accessible source for data mining. It should be noted in particular that by automating the connection between systems, and by providing all the information under a single umbrella and access system, not only is a wealth of standardized information available, but also (or) very little of the delay can be attributed to computerized systems or "mismatch" and that which is, can generally be measured. This allows allocating more exact measures to people.

For example, for a reading group, such data mining can allow comparing the group to other groups (and the readers, within a group), for example, with respect to time, delay, quality of diagnosis and abilities. In addition, the data mining can support better design of the workgroup and/or of load balancing and other procedures. Load balancing and/or remote reading may also reduce stress.

For example, for a payer, such as an HMO or a hospital, it is now possible to track various procedures form start to finish, with regard to one or more of efficacy, cost, degree of information provided, effect on morbidity, patient/physician satisfaction, usage of alternative imaging techniques, quality of reading and/or quality of scanning.

For example, for a health professional, such as a medical director, it is possible to design new procedures for handling various diseases and see the effect of such procedures of past data (e.g., by spread sheet analysis) or future data (e.g., as such data arrives). Optionally or alternatively, such a health director can evaluate doctors, such as referring physicians and doctors, since a complete life cycle can now be available, including complaints and suggestions.

For example, for an imaging service provided, it is possible to see what imaging systems are used for and if they meet their purpose. Also, it is possible to better match the imaging systems to the available readers, possibly suggesting the obtaining of additional reading services and/or defining expected turn-around times.

In an exemplary embodiment of the invention, data is compared between sites that are not affiliated with each other, possibly with a hiding of the data source or the use of industry averages.

In an exemplary embodiment of the invention, a radiologist will be able to specify a feedback on the quality of the scan/reconstructions done by the technician. This information can be used to track down bottlenecks and provide feedback to the technicians.

In an exemplary embodiment of the invention, a radiologist will be able to perform quality assurance on the prior exams of the patient he is reviewing. For example the radiologist can read the previous report and state if he agrees or not, or otherwise provide a second opinion. This can also be used to track the quality of reading.

In an exemplary embodiment of the invention, the system can calculate statistics on how long it takes a radiologists to read a case, identifying high/low performers.

In an exemplary embodiment of the invention, the system can calculate statistics on an average patient turn-around, providing feedback to organizational changes targeted for efficiency improvements.

In an exemplary embodiment of the invention, the system can be used as a scientific data-mining tool for gathering statistics, for example, percentage of women over 60 with breast cancer or percentage of lesions detected in virtual colonoscopy (optionally in comparison with regular colonoscopy, which is optionally retrieved using a "pre-fetch" function).

### General

While the above description has focused on imaging radiology, the above systems and methods may also be used for interventional radiology, in some cases, bypassing the diagnosis activities. Optionally or alternatively, the above systems and methods are used for non-radiology applications, for example, integration device 300 including a HIS system or a laboratory results systems (or other hospital information system) instead of or in addition to a PACS system and radiology related interfaces.

It is expected that during the life of a patent maturing from this application many relevant hospital information systems will be developed and the scope of the term data is intended to include all such new technologies *a priori.*

As used herein the term "about" refers to ± 10 %.

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

## Claims

1. A method of linking together a plurality of medical sites, selected as one or more instances of one or more elements of the following group of an imaging site, a hospital, a clinic, a reading center and a data center, comprising:
(a) installing an integration device at each of the sites;
(b) linking at least one integration device to a plurality of disparate medical information systems at one of the sites, to allow access to data therefrom; and
(c) exposing said data from said one integration device via the other integration device to a consumer of the data.

2. A method according to claim 1, wherein exposing comprises reading and updating.

3. A method accord to claim 1, wherein said linking comprises at least one of collecting meta data regarding said data at said one integration device and receiving at least an indication of said data without being allowed direct access to said data.

4. A method according to claim 1, comprising diagnosing on a single worklist and workstation studies from said plurality of sites by linking to one of said integration devices.

5. A method according to claim 1, wherein at least two of said disparate systems are incompatible with each other.

6. A method of upgrading a hospital information system infrastructure, comprising:
(a) installing an integration device in the hospital;
(b) linking said device to a plurality of hospital information systems and collecting at least meta data on data stored therein; and
(c) accessing data on a plurality of said systems via said integration device.

7. A method according to claim 6, comprising providing reliability redundancy using said integration device.

8. A method according to claim 6, comprising at least one of installing at least one additional integration device which is at least partially functionally redundant with said integration device, adding functionality of a type associated with one of said systems using said device, recovering from a failure using said device and one or both of meta data and data stored thereon, and changing a workflow in a site of said infrastructure by configuring said integration device.

9. A method according to claim 6, comprising data mining data across sites by combining data via said integration device and an integration device at another site.

10. A method of managing a radiological reading group, comprising:
(a) collecting studies from a plurality of sites not on a shared PACS system;
(b) arranging said studies into a single worklist; and
(c) managing said worklist across a plurality of readers located at a plurality of sites using an online computer system.

11. A method according to claim 10, comprises at least one of automatically updating said worklist within a time frame of less than 15 minutes, accessing and diagnosing a study at a site not affiliated with the study, in response to said worklist, and tracking availability of said readers using a computer.

12. A method of radiological diagnosis, comprising:
(a) diagnosing a study; and
(b) generating a report on a same online computer system as includes a RIS and a PACS.

13. A method according to claim 12, wherein said online computer system links a referring physician, a reader and a reporting system.

14. An integration device, comprising:
(a) a remote access module, configured to support remote clients;
(b) a data management module configured to manage data on the device and off of the device;
(c) at least one of a PACS functionality and a RIS functionality; and
(d) an integration module configured to at least one of link said device to a plurality of disparate hospital systems and link said device to an integration device at an additional site, for data sharing therewith.

15. A device according to claim 14, wherein said remote access module installs a complete suite of medical image processing and reporting software on a client device.

## Amended claims

### Amended claims in accordance with Rule 137(2) EPC.

1. A method of linking together a plurality of disparate medical information systems located in a plurality of medical sites, selected as one or more instances of one or more elements of the following group of an imaging site, a hospital, a clinic, a reading center and a data center, comprising:
(a) installing a plurality of integration devices at a plurality of medical sites;
(b) linking each said integration device to a plurality of disparate medical information systems installed at a respective said medical site;
(c) using said integration device to acquire data stored in a first of said a plurality of disparate medical information systems at a first said medical site by emulating a respective system operation;
(d) establishing at least one connection among said plurality of integration devices so as to create an inter-medical site network;
(e) transferring said data to a second of said plurality of disparate medical information systems at a second said medical site via at least one of said at least one connection; and
(f) providing said data to a customer via said second medical information system.

2. A method according to claim 1, wherein said providing comprises allowing said user to read and update said data.

3. A method accord to claim 1, wherein said linking comprises at least one of collecting meta data regarding said data at said one integration device and receiving at least an indication of said data without being allowed direct access to said data.

4. A method according to claim 1, comprising diagnosing on a single worklist and workstation studies from said plurality of sites by linking to one of said integration devices.

5. A method according to claim 1, wherein at least two of said disparate systems are incompatible with each other.

6. A method according to claim 1, wherein said data is a medical study; further comprising allowing said user to diagnose said study and generating a report according to said diagnose.

7. A method according to claim 6, further comprising linking said report to a referring physician, a reader and a reporting system.

8. A method according to claim 1, wherein said data comprises radiological imaging data and one of said medical information systems is a PACS system.

9. A method according to claim 1, comprising diagnosing on a single worklist and workstation studies from said plurality of sites by linking to one of said integration devices.

10. A method of upgrading a hospital information system infrastructure, comprising:
(a) installing an integration device in the hospital;
(b) linking said integration device to a plurality of hospital information systems and using said integration device for collecting at least meta data on data stored therein by emulating a respective system operation; and
(c) accessing data on a plurality of said systems via said integration device using said at least meta data.

11. A method according to claim 10, comprising providing reliability redundancy using said integration device.

12. A method according to claim 10, comprising at least one of installing at least one additional integration device which is at least partially functionally redundant with said integration device, adding functionality of a type associated with one of said systems using said device, recovering from a failure using said device and one or both of meta data and data stored thereon, and changing a workflow in a site of said infrastructure by configuring said integration device.

13. A method according to claim 10, comprising data mining data across sites by combining data via said integration device and an integration device at another site.

14. A method of managing a radiological reading group, comprising:
(a) collecting studies from a plurality of disparate medical systems at a plurality of medical sites;
(b) arranging said studies into a single worklist; and
(c) managing said worklist across a plurality of readers located at a plurality of client sites using an online computer system.

15. A method according to claim 14, comprises at least one of automatically updating said worklist within a time frame of less than 15 minutes, accessing and diagnosing a study at a site not affiliated with the study, in response to said worklist, and tracking availability of said readers using a computer.
